# EUROPEAN PATENT APPLICATION

(11) **EP 4 074 467 A2**
(43) Date of publication of application: **19.10.2022**
(21) Application number: 20900579.2
(22) Date of filing: 07.12.2020
(51) Int. Cl.: B25J 9/10, B25J 9/00, B25J 19/00, B25J 19/02

(54) **SOFT ACTUATOR, SOFT ACTUATOR ASSEMBLY COMPRISING SOFT ACTUATOR, AND WEARABLE ROBOT COMPRISING SOFT ACTUATOR OR SOFT ACTUATOR ASSEMBLY**

(30) Priority: 13.12.2019 KR 20190166510; 21.05.2020 KR 20200060752
(71) Applicant: Korea Institute of Machinery & Materials, Daejeon 34103 (KR)
(72) Inventor: PARK, Cheol-hoon, Daejeon 34022 (KR); KIM, Seyoung, Anyang-si Gyeonggi-do 13953 (KR); SONG, Sung-Hyuk, Daejeon 34070 (KR); SEO, Hyunuk, Seoul 08711 (KR); HAM, Sang-Yong, Daejeon 34209 (KR); JUNG, Hyun Mok, Sejong 30150 (KR); KIM, Byung In, Sejong 30151 (KR); PARK, Seongjun, Daejeon 34199 (KR); CHOI, Kyungjun, Daejeon 35040 (KR)
(74) Representative: Lewis Silkin LLP
(86) International application number: PCT/KR2020/017746
(87) International publication number: WO 2021/118185

(57) **Abstract**

In a soft actuator, a soft actuator assembly having the soft actuator, and a wearable robot having the soft actuator or the soft actuator assembly, the soft actuator includes a first spring bundle, a first conductive pad and a second conductive pad. The first spring bundle has a plurality of fine wires, and is configured to be capable of being changed between a contraction state and a relaxation state according to a change of temperature. The first conductive pad has a first connector electrically connected to a first end of the first spring bundle. The second conductive pad has a second connector electrically connected to a second end of the first spring bundle. The first connector is fixed between the first conductive pad and the first spring bundle, and the second connector is fixed between the second conductive pad and the first spring bundle.

## Description

### BACKGROUND

### 1. Field of Disclosure

The present disclosure of invention relates to a soft actuator, a soft actuator assembly having the soft actuator, and a wearable robot having the soft actuator or the soft actuator assembly, and more specifically the present disclosure of invention relates to a soft actuator, a soft actuator assembly having the soft actuator, and a wearable robot having the soft actuator or the soft actuator assembly, in which the soft actuator is manufactured by a spring bundle having fine wires and is fixed to materials such as cloth.

### 2. Description of Related Technology

Recently, the need for a wearable muscle support device is emerging to improve the work efficiency of workers working in various industrial fields and to improve the safety of the workers.

However, most recently developed muscle support devices are driven by attaching the devices to arms or legs using motors and frames, and those devices have the motors and frames and thus have a large weight. In addition, those devices also have a heavy structure and a natural movement of the worker is interfered and to wear the devices is uncomfortable to the worker.

Thus, it is necessary to develop a wearable robot (clothes for strengthening muscles) that is light in weight, attached to a position similar to the human muscle, does not interfere with the movement of the body of the worker, and improves the responsiveness of various movements. Thus, studies on a thermally responsive driving device capable of converting thermal energy into mechanical energy such as driving force or displacement have been conducted.

Among the thermally responsive driving devices, a shape memory alloy (hereinafter, SMA) is a material that is restored to its original shape, when it is deformed by applying stress to the material in the low temperature martensitic state and then heated to the high temperature austenitic state, as disclosed in Korean laid-open patent No. 10-2010-0137235. Thus, when the SMA wire with a contraction displacement of 2~5% is manufactured as a spring, the contraction displacement may be improved to more than about 40%.

However, the SMA spring is heated and contracted quickly, but the cooling rate of the SMA spring is slow, so that to improve the relaxation rate of the SMA spring is limited. Thus, an entire driving velocity of the SMA spring may be decreased.

Further, as for a soft actuator having the conventional SMA spring, when the soft actuator is fixed to a flexible material such as the cloth, each spring should be fixed to each cloth, a power line for applying a current to the spring should be formed, and an insulation for the power line should be designed. Thus, the structure may be complicated and the manufacturing may be difficult.

### SUMMARY

The present invention is developed to solve the above-mentioned problems of the related arts. The present invention provides a soft actuator attached to a position similar to the human muscle, capable of not interfering with the movement of the body but also performing various movements, and capable of maintaining a fit and improving the responsiveness of relaxation movements,

In addition, the present invention also provides a soft actuator assembly having the soft actuator.

In addition, the present invention also provides a wearable robot having the soft actuator or the soft actuator assembly.

According to an example embodiment, the soft actuator includes a first spring bundle, a first conductive pad and a second conductive pad. The first spring bundle has a plurality of fine wires, and is configured to be capable of being changed between a contraction state and a relaxation state according to a change of temperature. The first conductive pad has a first connector electrically connected to a first end of the first spring bundle. The second conductive pad has a second connector electrically connected to a second end of the first spring bundle. The first connector is fixed between the first conductive pad and the first spring bundle, and the second connector is fixed between the second conductive pad and the first spring bundle.

In an example, the first connector may be fixed between the first conductive pad and the first spring bundle via sewing, and the second connector may be fixed between the second conductive pad and the first spring bundle.

In an example, each of the fine wires may be a shape memory alloy which is driven according to the change of temperature.

In an example, each of the first and second conductive pads may have a foldable conductive material.

In an example, the soft actuator may further include an outer cover having a flexible material, and having the first spring bundle inside of the outer cover. The first and second conductive pads may be fixed to the outer cover.

In an example, the soft actuator may further include an additional fabric integrally fixed to the first and second connectors, and having a material less flexible than the outer cover.

In an example, each of the first and second connectors may be folded at least one time, and the additional fabric may be inserted into and fixed to a folded portion of each of the first and second connectors.

In an example, the additional fabric may include an opening portion for an eyelet.

In an example, the temperature may change according to a current supplied by outside.

According to another example embodiment, a soft actuator includes spring bundles and electrodes. The spring bundles have a first spring bundle and a second spring bundle, and are configured to be capable of being changed between a contraction state and a relaxation state according to a change of temperature. Each of the first and second spring bundles has a plurality of fine wires. The electrodes are electrically connected to the first and second spring bundles, and have a first conductive pad, a middle conductive pad and a second conductive pad. The first conductive pad has a first connector electrically connected to a first end of the first spring bundle. The second conductive pad has a second connector electrically connected to a second end of the second spring bundle. The middle conductive pad has a middle connector electrically connecting the first and second spring bundles with each other.

In an example, the spring bundles may further include at least one middle spring bundle disposed between the first spring bundle and the second spring bundle. The middle spring bundle may include a plurality of the middle connectors.

In an example, the soft actuator may further include an external current receiver formed from the first conductive pad, and configured to receive a current from outside to form an electric path between the spring bundles and the electrodes.

According to still another example embodiment, a wearable robot includes the soft actuator, a sensor and a controller. The sensor is configured to sense a motion of a user. The controller is configured to control the contraction state or the relaxation state of the soft actuator, based on a signal of the sensor. The soft actuator is disposed at a flexor or an extensor.

According to still another example embodiment, a soft actuator assembly includes a first and second soft actuators, a cool air supplier and a controller. The first and second soft actuators are configured to be capable of being changed between a contraction state and a relaxation state according to a change of temperature. The cool air supplier is configured to connect the first and second soft actuators with each other to pass through an air, and is configured to make the air inflow into a relaxed soft actuator of the first and second soft actuators. The controller is configured to control the contraction state or the relaxation state of the first and second soft actuators.

In an example, each of the first and second soft actuators may include a first spring bundle, a first conductive pad and a second conductive pad. The first spring bundle may have a plurality of fine wires, and may be configured to be capable of being changed between the contraction state and the relaxation state according to a change of temperature. The first conductive pad may have a first connector electrically connected to a first end of the first spring bundle. The second conductive pad may have a second connector electrically connected to a second end of the first spring bundle. The first connector may be fixed between the first conductive pad and the first spring bundle, and the second connector may be fixed between the second conductive pad and the first spring bundle.

In an example, the cool air supplier may be operated without a power, and may be inflated or contracted by an external force.

In an example, the cool air supplier may include a check valve disposed at each of the first and second soft actuators to control a flow direction of the air. The check valve at the relaxed soft actuator of the first and second soft actuators may be open, and the check valve at a contracted soft actuator of the first and second soft actuators may be closed.

In an example, each of the first and second soft actuators may include a thermal reactive member configured to be capable of being changed between the contraction state and the relaxation state according to a change of temperature. A cool air for cooling the thermal reactive member may make direct contact with the thermal reactive member to increase a relaxation speed of the thermal reactive member, when the thermal reactive member is in the relaxation state.

According to still another example embodiment, a wearable robot includes the soft actuator, and the cool air supplier is disposed at a joint, the first soft actuator is disposed at a flexor and a second soft actuator is disposed at an extensor.

In an example, the first soft actuator may be contracted and the second soft actuator may be relaxed, and then the cool air supplier may be contracted to move an inner air to the second soft actuator, when the joint moves from an extended state to a flexed state. The first soft actuator may be relaxed and the second soft actuator may be contracted, and then the cool air supplier may be inflated to move an external air to pass through the first soft actuator into the second soft actuator, when the joint moves from the flexed state to the extended state.

According to the present example embodiments, the thermal reactive member implemented as a spring with a fine wire having a fine diameter is used to manufacture the soft actuator and the wearable robot having the soft actuator. Thus, the existing driving force is maintained, the fabric is maintained to have a considerable part of the flexibility in wearing so that awkwardness may not be sensed, and the response performance is significantly improved.

In addition, compared to the conventional complicated manufacturing process, the process for manufacturing the soft actuator may be more simplified and may prevent wastage of materials.

Here, since the SMA spring has a relatively larger power density of the SMA wire and a relatively larger displacement is generated, for example about hundreds of percent (%), the soft actuator may be applied in small size, light weight and noiseless wearable type, and may have a quick response in the relaxation state, due to the cooling system using a self-air supplying type.

### BRIEF DESCRIPTION OF THE DRAWINGS

FIG. 1A is a plan view illustrating a soft actuator assembly having a soft actuator according to an example embodiment of the present invention, and FIG. 1B is an exploded perspective view illustrating the soft actuator of FIG. 1A;
FIG. 2A is an image showing a spring bundle of the soft actuator of FIG. 1A, and FIG. 2B and FIG. 2C are images showing each unit spring of the spring bundle of FIG. 2A;
FIG. 3A and FIG. 3B are a perspective view and a side view illustrating an additional fabric of the soft actuator FIG. 1A;
FIG. 4A and FIG. 4B are plan views illustrating soft actuators according to other example embodiments of the present invention;
FIG. 5A, FIG. 5B, FIG. 5C, FIG. 5D, FIG. 5E and FIG. 5F are process views illustrating a method for manufacturing the soft actuator of FIG. 1A;
FIG. 6 is a plan view illustrating a soft actuator assembly according to still another example embodiment of the present invention;
FIG. 7 is a schematic view illustrating one soft actuator of the soft actuator assembly of FIG. 6;
FIG. 8A, FIG. 8B, FIG. 8C and FIG. 8D are schematic views illustrating a contraction motion and a relaxation motion of the soft actuator of FIG. 7;
FIG. 9 is a plan view illustrating a cool air supplier of FIG. 6;
FIG. 10 is a schematic view illustrating an inflated state of the cool air supplier of the soft actuator of FIG. 6, and FIG. 11 is a schematic view illustrating a contracted state of the cool air supplier of the soft actuator of FIG. 6;
FIG. 12 is a schematic view illustrating a wearable robot according to still another example embodiment of the present invention;
FIG. 13A and FIG. 13B are schematic views illustrating an operation state of the wearable robot of FIG. 12;
FIG. 14A is a schematic view illustrating a joint flexion state of the wearable robot of FIG. 12, and FIG. 14B is a schematic view illustrating a joint unfolding state of the wearable robot of FIG. 12; and
FIG. 15A is a schematic view illustrating a joint flexion state with the user wearing the wearable robot of FIG. 12, and FIG. 15B is a schematic view illustrating a joint unfolding state with the user wearing the wearable robot of FIG. 12.

### ^{∗} Reference numerals

| | | | |
|---|---|---|---|
| 1, 2 : | soft actuator assembly | 10, 10a, 10b, 11, 12 : | soft actuator |
| 20 : | wearable robot | 30 : | cloth body |
| 100 : | thermal reactive member/spring bundle | | |
| 101 : | unit spring | 101a | : body portion |
| 101b: | leg portion | 110 : | first spring bundle |
| 120 : | second spring bundle | 130 : | middle spring bundle |
| 200 : | electrode/conductive pad | 210 : | first conductive pad |
| 211 : | first connector | 220 : | second conductive pad |
| 221 : | second connector | 230 : | middle conductive pad |
| 231 : | middle connector | 240 : | external current receiver |
| 300 : | controller | 400 : | outer cover/body |
| 410 : | cool air inlet | 420 : | cool air outlet |
| 500 : | additional fabric | 501 : | eyelet/opening portion |
| 600 : | cool air supplier | 610 : | air pump(or air bag) |
| 620 : | check valve | 630 : | cool air path |

### DETAILED DESCRIPTION

The invention is described more fully hereinafter with Reference to the accompanying drawings, in which embodiments of the invention are shown. This invention may, however, be embodied in many different forms and should not be construed as limited to the embodiments set forth herein. Rather, these embodiments are provided so that this disclosure will be thorough and complete, and will fully convey the scope of the invention to those skilled in the art. In the drawings, the size and relative sizes of layers and regions may be exaggerated for clarity.

It will be understood that, although the terms first, second, third etc. may be used herein to describe various elements, components, regions, layers and/or sections, these elements, components, regions, layers and/or sections should not be limited by these terms. These terms are only used to distinguish one element, component, region, layer or section from another region, layer or section.

The terminology used herein is for the purpose of describing particular embodiments only and is not intended to be limiting of the invention. As used herein, the singular forms "a," "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. Unless otherwise defined, all terms (including technical and scientific terms) used herein have the same meaning as commonly understood by one of ordinary skill in the art to which this invention belongs.

In the example embodiments of the present invention, as a diameter of a fine wire forming a SMA spring becomes thinner, a heating speed becomes faster and a ratio of a surface area with respect to a unit volume is increased and then a cooling speed is also increased.

For example, the fine wire having a diameter of about 0.08mm is about 1/39 of the fine wire having a diameter of about 0.5mm, and thus the ratio of the surface area with respect to the unit volume is increased by about 12.5 times. Since a load capacity is decreased as much as the ratio of the diameter, the SMA spring having the fine wire of about 0.08mm diameter is required to be 39 pieces theoretically, to have the load capacity substantially same as that of the SMA spring having the fine wire of about 0.5mm diameter.

When a fabric actuator is manufactured to have the driving force of about 10kgf with the SMA spring having the fine wires of about 0.5mm diameter, several numbers (for example, 20 pieces) of SMA springs may be required, but the user may feel uncomfortable and awkward in wearing the fabric actuator having the several numbers of the SMA springs.

In addition, when the SMA spring having the fine wires of about 0.08mm diameter is used for the fabric actuator, hundreds of the SMA springs should be used to provide the driving force of about 10kf of the fabric actuator mentioned above.

Thus, in the present example embodiments, to solve the above problem, the SMA spring having a lot of fine diameter wires is used to manufacture the soft actuator, the soft actuator assembly and the wearable robot more efficiently and more simply.

Hereinafter, example embodiments of the present invention are explained in detail referring to the figures.

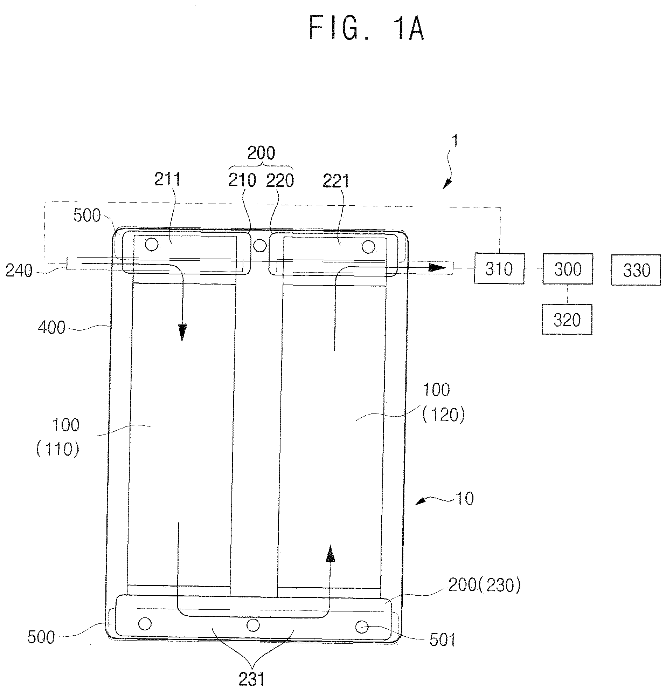

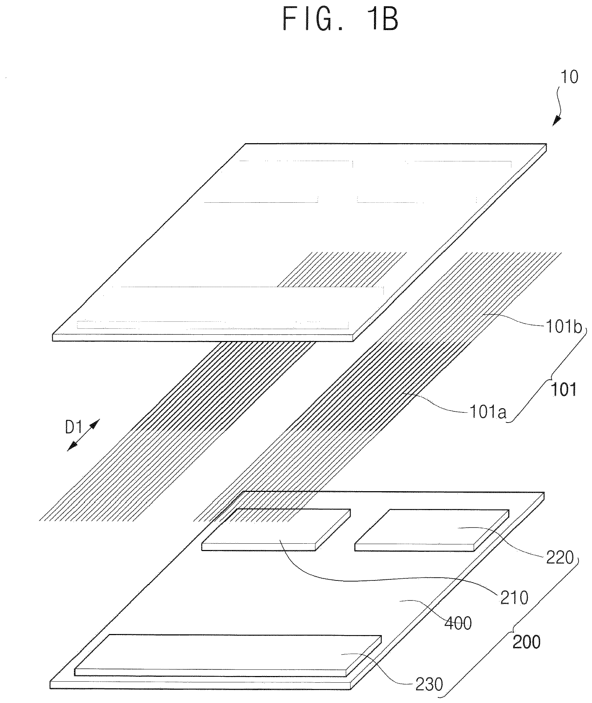

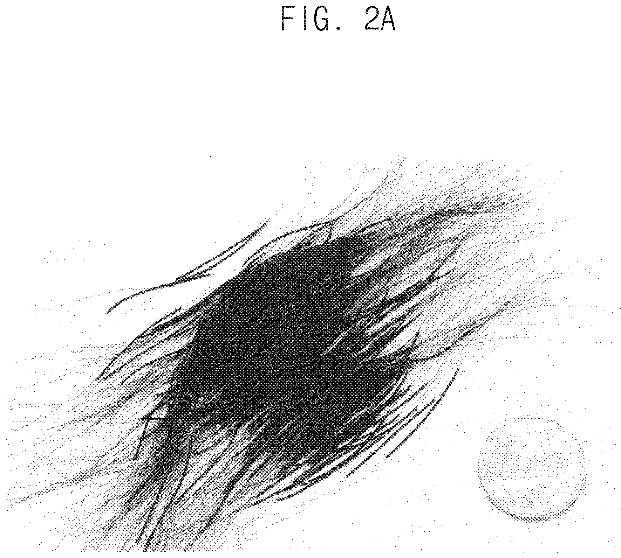

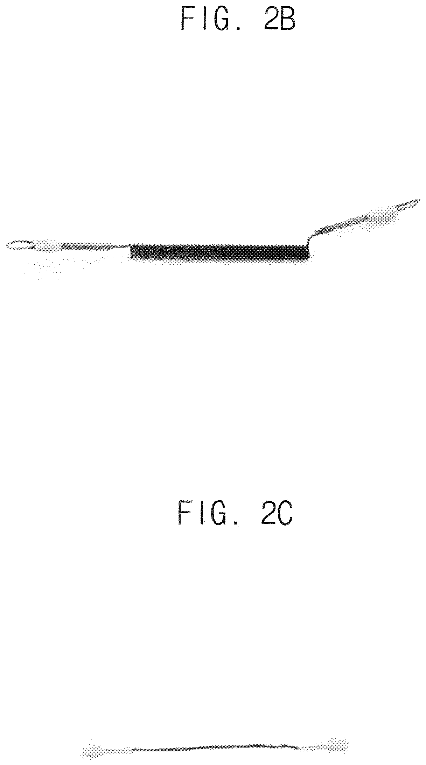

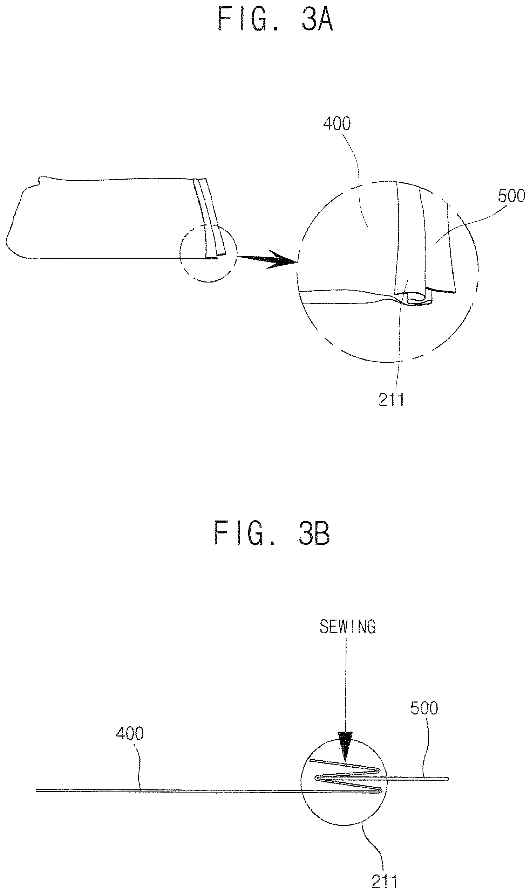

FIG. 1A is a plan view illustrating a soft actuator assembly having a soft actuator according to an example embodiment of the present invention, and FIG. 1B is an exploded perspective view illustrating the soft actuator of FIG. 1A. FIG. 2A is an image showing a spring bundle of the soft actuator of FIG. 1A, and FIG. 2B and FIG. 2C are images showing each unit spring of the spring bundle of FIG. 2A. FIG. 3A and FIG. 3B are a perspective view and a side view illustrating an additional fabric of the soft actuator FIG. 1A.

Referring to FIG. 1A and FIG. 1B, the soft actuator assembly 1 according to the present example embodiment includes a soft actuator 10, a controller 300, a power supplier 310, a sensor 320 and a power source 330. The controller 300 is configured to control the power supply for changing the operation state between a relaxation state and a contraction state of the soft actuator 10.

The controller 300 is configured to control the power supply provided to a thermal reactive member 100, so that the thermal reactive member (spring bundle, 100) of the soft actuator 10 is changed between the relaxation state and the contraction state.

For example, the controller 300 controls the power supply to the thermal reactive member 100, using a power supplier 310. When the controller 300 transmits a power supply signal to the power supplier 310, the power supplier 310 supplies a current to the thermal reactive member 100. In addition, when the controller 300 stops transmitting the power signal to the power supplier 310, the power supplier 310 stops supplying the current to the thermal reactive member 100.

Accordingly, as the current is applied to the thermal reactive member 100, the heat is generated to contract the heat reactive member 100. As the current is not applied to the thermal reactive member 100, the temperature is decreased to relax the heat reactive member 100.

The power supplier 310 may be configured to supply the current to the heat reactive member 100, and further detailed explanation for the power supplier is omitted.

The sensor 320 may include various kinds of devices configured to sense a bio-information, a motion and son on of a user. Here, the bio-information may include electromyography information.

For example, when the sensor 320 may include an electromyography sensor, the sensor may sense a motion (a contraction motion or a relaxation motion) of a muscle of the user as the user grips, moves and supports a load. Alternatively, the sensor 320 may include a voice sensor, and then the sensor may be configured to receive an action, a state, a request of the user via a vice information of the user. Here, the user means a wearer wearing the soft actuator.

The sensor 320 may be configured to measure a strain of the thermal reactive member 100, and for example, the sensor 320 may be a strain gauge.

The power source 330 is configured to supply the power to at least one of the controller 300, the power supplier 310 and the sensor 320.

The controller 300 controls the current from the power supplier 310 to the thermal reactive member 100, based on the information from the sensor 320.

For example, when the user performs the motion requiring a contraction of the thermal reactive member like an arm bending motion, the sensor 320 transmits the measured electromyography information to the controller 300. Then, the controller 300 detects the user to intend to bend the arm, based on the electromyography information. In addition, the controller 300 calculates a force for bending the arm, to obtain a target force outputted from the soft actuator 10. Further, the controller 300 may control the current from the power supplier 310 to the thermal reactive member 100, to output the target force at the soft actuator 10.

Referring to FIG. 2A, FIG. 2B and FIG. 2C additionally, in the present example embodiment, the soft actuator 10 is configured to be contracted and expanded, as the thermal reactive member 100 which is the spring bundle is contracted or expanded by the controller 300.

The thermal reactive member 100 which is the spring bundle extends along a direction D1, and may be configured to be contracted or relaxed along the direction D1. For example, the thermal reactive member 100 may be contracted along the direction D1, in reaction to the heat generated by the current, and may be relaxed along the opposite direction, as the temperature is decreased without the current.

The thermal reactive member 100 may include a shape memory alloy (SMA). For example, the thermal reactive member 100 may include a shape memory alloy spring which is performed by the shape memory alloy fine (or micro) wire.

Alternatively, the thermal reactive member 100 may include various kinds of thermal reactive materials such as a shape memory resin, a shape memory polymer (SMP), a carbon nanotube, polyethylene, polyamide, nylon and so on.

Hereinafter, as for the thermal reactive member 100, the shape memory alloy fine wire implemented as the SMA spring is explained as an example, but the materials are not limited thereto. The SMA unit spring 101 includes a changeable body portion 101a and a leg portion 101b for the coupling.

Here, in FIG. 2B and FIG. 2C, the diameter of the wire for the spring is exampled to be about 0.5mm and 0.08mm, but not limited thereto. For example, the wire having the diameter larger than about 0.5mm may be applied, and any wire of folded diameter may be applied.

For example, hundreds of the fine SMA springs are collected to form a spring bundle 100 which is a single module. When the fine wire based bundle having the fine diameter is used, the existing driving force is maintained, the fabric is maintained to have a considerable part of the flexibility in wearing so that awkwardness may not be sensed in wearing, and the response performance is significantly improved via increasing a cooling speed. In addition, the number of the springs forming the bundle is not limited, and thus several numbers to hundred numbers of springs may be formed as a single bundle.

The SMA spring may be manufactured from the technical features disclosed in Korean laid-open number 10-2021-0114180 (Method of manufacturing shape memory alloy spring), which is incorporated herein by reference in its entirety. However, the method for manufacturing the SMA spring may not be limited to the method above.

The fine wire based bundle having the fine diameter may be a single or plural according to the driving force required.

In the present example embodiment, the SMA unit spring 101 based on the fine wire having the fine or predetermined diameter is configured to be the plural having a first spring bundle 110 and a second spring bundle 120, and as illustrated in FIG. 1A and FIG. 1B, the first and second spring bundles 110 and 120 may be disposed in parallel and thus may be contracted or relaxed along the same direction.

The soft actuator 10 includes an outer cover 400, a spring bundle 100, 110 and 120, a conductive pad which is an electrode 200, and an external current receiver 240.

The outer cover 400 includes a pair of upper cover and lower cover. The outer cover 400 may include various kinds of materials, and include a material contracted or relaxed with the contraction or the relaxation of the spring bundle. For example, the outer cover 400 may include a flexible material or a fabric material.

The spring bundle 100 is a module formed by several numbers or hundreds numbers of fine wires having the fine diameter. The spring bundle 100 is configured to be capable of being changed between the relaxation state and the contraction state, in the outer cover 400, according to a change of temperature.

In terms of current flow, the plurality of the spring bundles 100 includes a first spring bundle 110 disposed at a front end which is at an uppermost portion, and a second spring bundle 120 disposed at a rear end which is at a lowermost portion. Each of the spring bundles 110 and 120 includes an inlet first end through which the current enters and an outlet second end through which the current goes out.

In the present example embodiment, as illustrated in FIG. 1A, the first spring bundle 110 and the second spring bundle 120 are arranged in parallel, and thus are contracted or relaxed along the same direction.

The electrode 200 (conductive pad) is a conductive fabric or a flexible conductive thin-film which acts as the electrode creating an electric path for the externally supplied current to flow to the spring bundle, and at least two electrodes may be formed. The conductive pad 200 may be disposed and fixed to the outer cover, via various kinds of methods.

In the present example embodiment, the electrode 200, in terms of current flow, includes a first conductive pad 210 disposed at the front end which is at the uppermost portion, a middle conductive pad 230, and a second conductive pad 220 disposed at the rear end which is at the lowermost portion.

The electrodes 200 have an area making contact with each of the spring bundles, to form the electric path with each of the spring bundles. The area is defined as a connector, and the connector may be a portion or an entire of the conductive pad. The conductive pad and the spring bundle are sewed and fixed with each other, to form the connector.

The first conductive pad 210 includes a first connector 211 electrically connected to a first end of the first spring bundle 110, and is formed at an upper portion of the outer cover 400 as illustrated in FIG. 1B.

The middle conductive pad 230 is formed at a lower portion of the outer cover 400, as illustrated in FIG. 1B, and thus the first spring bundle 110 and the second spring bundle 120 are electrically connected to each other. The middle conductive pad 230 includes two middle conductive pads 231 making contact with the first and second spring bundles 110 and 120, respectively.

The second conductive pad 220 includes a second connector 221 electrically connected to a second end of the second spring bundle 120, and is formed parallel with the first conductive pad 210 at the upper portion of the outer cover 400 as illustrated in FIG. 1a.

In terms of the direction in which the external current flows, the first conductive pad 210 is a front electrode which provides the external current to the first spring bundle disposed at the uppermost portion, and the second conductive pad 220 is a rear electrode which provides the current from the second spring bundle disposed at the lowermost portion to outside, and the middle conductive pad 230 is a middle electrode through which the current flows among the spring bundles.

As explained below, the middle electrode may be omitted when the spring bundle is one, may be two when the spring bundles are two, may be three when the spring bundles are three, or may be more.

As explained above, the connector means the area in which the unit spring forming the spring bundle is physically and electrically connected to the conductive pad, and may be at least portion of the conductive pad. Here, the leg portion 101b of the SMA spring is disposed at the connector, and thus the leg portion 101b is fixed and coupled with the conductive pad 200 via the sewing and so on.

Referring to FIG. 3A and FIG. 3B, each of the connectors may be folded with at least one time. In the conventional soft actuator using a relatively thicker wire, the leg of the SMA spring is sewn several times to highly rigid flexible plastic, to support external load. Here, to support the external load, the leg of the SMA spring is formed not to be pulled out from the plastic, and thus the process may be complicated.

However, in the present example embodiment, the legs of many SMA springs having the fine diameter are fixed to the conductive pad which is the electrode 200 and are folded at least one time. Then, the folded portion is formed with a 'Z' or similar shape, and the folded portion is fixed with the sewing. Thus, the legs of the SMA springs are not pulled out with the high external load. Accordingly, the manufacturing process may be simplified with the single sewing process mentioned above.

The soft actuator according to the present example embodiment, as illustrated in FIG. 3A and FIG. 3B, further includes an additional fabric 500. The additional fabric 500 is inserted into and integrally fixed to the folded portion as the 'Z' shape.

The additional fabric 500 may include a material less flexible than the outer cover. The additional fabric 500 may include an opening portion 501 for an eyelet, and the soft actuator may be connected with other elements through the opening portion 501. The additional fabric 500 may be a fabric having relatively higher rigidity like a bag strap, for fixing the eyelet and transmitting the force from outside.

The external current receiver 240 is a wire or a conductive fabric exposed outwardly to receive the current from outside into the inside of the outer cover. The external current receiver 240 is configured to form the electric path between the spring bundles and the conductive pads of FIG. 1A, to receive the current from outside for the first time.

Here, the external current receiver 240 is electrically connected to the power supplier 310 explained above, and receives the current from the power supplier 310.

In the present example embodiment, the temperature of the soft actuator 10 is changed as the current is provided from the power supplier 310, but the temperature of the soft actuator 10 may be changed due to other configurations.

The external current receiver 240 may be formed from the first conductive pad 210 which is the front electrode at the uppermost portion. Conventionally, the leg of the SMA spring sewn to the flexible plastic is pulled out and is used for the current supplier, but in the conventional technology, the length of the leg of the SMA spring should be long and thus the material may be wasted and the process may be complicated.

In contrast, in the present example embodiment, the conductive pad of the conductive fabric used for the electrode is used for the current path, and thus the conductive fabric is extended outwardly to be used for the current supplier. Thus, the leg of the SMA spring may be formed short and uniform, and the material may be prevented from being wasted and the process may be more simplified.

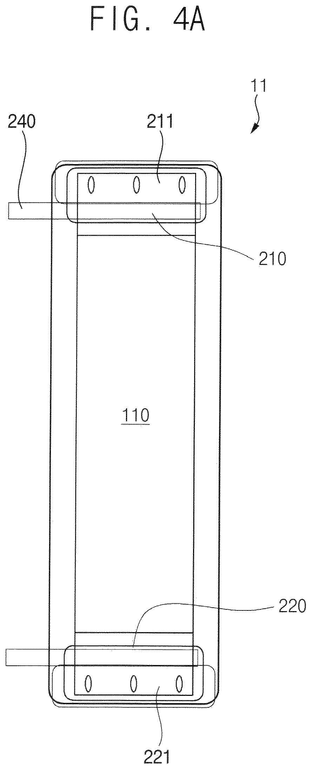

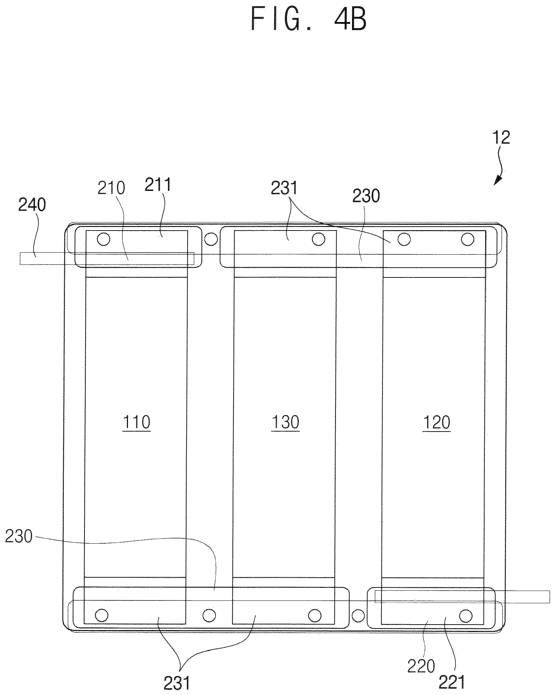

FIG. 4A and FIG. 4B are plan views illustrating soft actuators according to other example embodiments of the present invention.

Referring to FIG. 4A, the soft actuator 11 according to the present example embodiment includes a single spring bundle 110.

Thus, as explained above, in the soft actuator 11 according to the present example embodiment, in terms of the direction of the external current, the first conductive pad 210 and the second conductive pad 220 are necessary and the middle electrode is not required. Here, the first conductive pad 210 which is the front electrode transmits the external current to the first spring bundle which is disposed at the uppermost portion. The second conductive pad 220 which is the rear electrode transmits the current to outside.

In the present example embodiment, the soft actuator 11 includes a pair of outer covers having flexible materials, the first spring bundle 110, the first conductive pad 210, the second conductive pad 220 and the external current receiver 240. The first spring bundle 110 has a plurality of fine wires, and is configured to be capable of being changed between a contraction state and a relaxation state according to a change of temperature, inside of the outer cover. The first conductive pad 210 has a first connector 211 fixed at the outer cover and electrically connected to a first end of the first spring bundle 110. The second conductive pad 220 has a second connector 221 fixed at the outer cover and electrically connected to a second end of the first spring bundle 110. The external current receiver 240 is configured to receive the current from outside, to form the electric path from the first conductive pad 210 and the first spring bundle 110 to the second conductive pad 220. Here, each of the first and second connectors 211 and 221 is sewed.

Here, the soft actuator 11 according to the present example embodiment is substantially same as the soft actuator 10 in FIG. 1A to FIG. 3B, except for the above mentioned configurations, and thus any repetitive explanation will be omitted.

Referring to FIG. 4B, the soft actuator 12 according to the present example embodiment, includes a plurality of spring bundles 110, 120 and 130.

Here, in FIG. 4B, three spring bundles are illustrated, but the number of the spring bundles is not limited thereto. Hereinafter, for the convenience of explanation, three spring bundles 110, 120 and 130 are explained.

In the present example embodiment, the soft actuator 12 includes the first conductive pad 210 which is the front electrode, the second conductive pad 220 which is the rear electrode, and the middle pads 230 which is the middle electrode for electrically connecting the first to third spring bundles 110, 120 and 130 with each other.

Thus, the electrodes of the soft actuator 12 include the plurality of middle conductive pads 230 and the plurality of middle connectors 231.

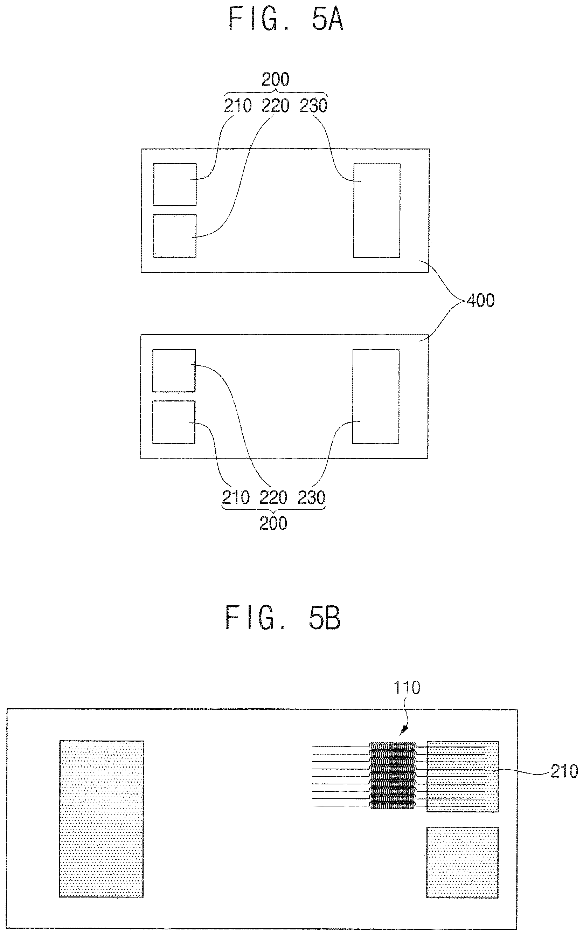

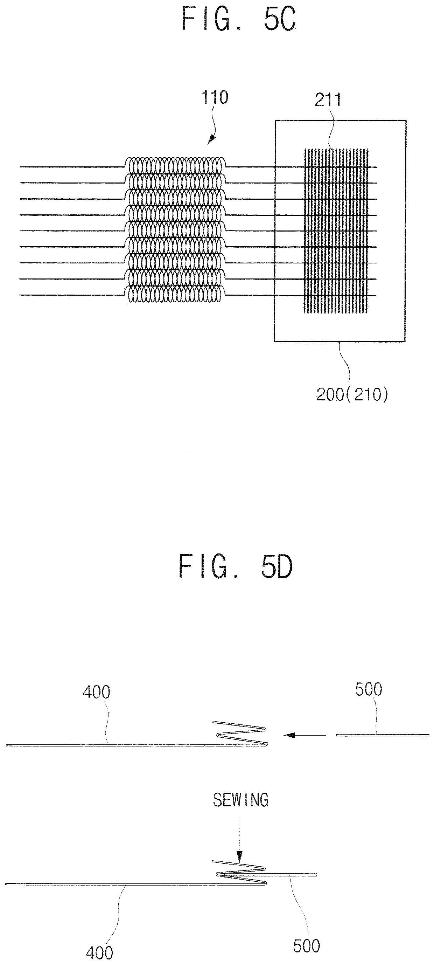

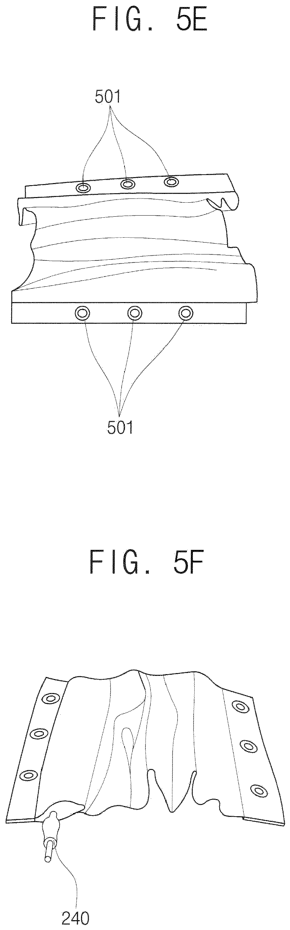

The soft actuator 12 according to the present example embodiment is substantially same as the soft actuator 10 in FIG. 1A to FIG. 3B and the soft actuator 11 in FIG. 4A, except for the configurations of the soft actuator 12 explained above, and thus any repetitive explanation will be omitted. FIG. 5A, FIG. 5B, FIG. 5C, FIG. 5D, FIG. 5E and FIG. 5F are process views illustrating a method for manufacturing the soft actuator of FIG. 1A.

In the method for manufacturing the soft actuator according to the present example embodiment, the pair of outer covers 400 having the upper cover and the lower cover with the flexible material is prepared. The electrode 200 having the plurality of conductive pads 210, 220 and 230 is formed on at least one outer cover 400, referring to FIG. 5A. At least one spring bundle 100 having the fine wires is disposed such that the first and second ends of the spring bundle 100 are formed to be the connector 211 electrically connected to the conductive pad 200, referring to FIG. 5B. The connector 211 is sewed, referring to FIG. 5C. The external current receive 240 is formed to receive the external current and to form the electric path between the electrode 200 and the spring bundle 100, referring to FIG. 5F.

More specifically, referring to FIG. 5A, the pair of the outer covers 400 having the upper cover and the lower cover with the flexible material is prepared. The plurality of electrodes 200, for example, the first conductive pad 210 at the front end, the middle pad 230, and the second conductive pad 220 at the rear end, is formed on at least one of the pair of the outer covers 400. For example, the conductive fabric which is the conductive pad may be formed on the upper cover, the lower cover or both of the upper and the lower covers, by the sewing.

Then, referring to FIG. 5b, the leg portion which is the first end of each of the first spring bundle 110 having the fine wires is positioned on the electrode of the lower cover, and then the first connector 211 is formed to be electrically connected with the first conductive pad 210. The same process may be applied to form the second connector 221. Then, the leg portion which is the second end of each of the second spring bundle 120 having the fine wires is positioned on the electrode of the lower cover, and then the second connector 221 is formed to be electrically connected with the second conductive pad 220.

Referring to FIG. 5C, the connectors 211 and 221 are sewed and fixed with the conductive pads 210 and 220 respectively corresponding to the first and second ends of the spring bundles 110 and 120 at the same time.

Then, the upper cover is disposed on the lower cover, and then the upper and lower covers are sewed and coupled with each other.

Then, the sewing may be performed again, with the first and second connectors are folded at least one time, and then, as illustrated in FIG. 5D, the folded portion of the first and second connectors may have the 'Z' shape with the folding of two times.

Here, the sewing is performed with the additional fabric 500 inserted into the folded portion of the first and second connectors, to fix the additional fabric 500 with the connectors, and then the first spring bundle 110 may be prevented from being detached from the conductive pad. Here, the additional fabric 500 may include a material less flexible than the outer cover. In addition, the opening portion may be formed at the end of the additional fabric 500 and the eyelet 501 may be formed at the opening portion, as illustrated in FIG. 5E.

Finally, referring to FIG. 5F, the external current receiver 240 which receives the current from outside is formed, to form the electric path through the first conductive pad 210, the first spring bundle 110 and the second conductive pad 220. The external current receive 240 may be formed from the first conductive pad 210, as explained above.

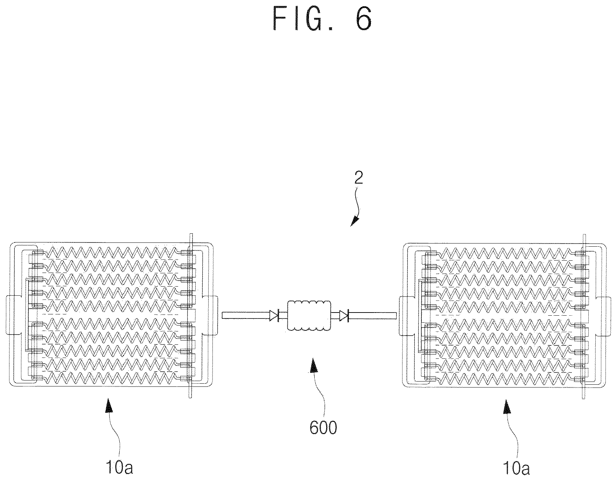

FIG. 6 is a plan view illustrating a soft actuator assembly according to still another example embodiment of the present invention.

Referring to FIG. 6, the soft actuator assembly according to the present example embodiment includes a first soft actuator 10a, the second soft actuator 10b, a cool air supplier 600 and a controller 300. The first and second soft actuators 10a and 10b are operated between the relaxation state and the contraction state. The cool air supplier 600 is disposed between the first and second soft actuators 10a and 10b and the cool air flows into the first and second soft actuators 10a and 10b. The controller 300 controls the power supply to operate the first and second soft actuators 10a and 10b between the relaxation state and the contraction state.

Here, each of the first and second soft actuators 10a and 10b may be one of the soft actuator 10 in FIG. 1A, the soft actuator 11 in FIG. 4A, and the soft actuator 12 in FIG. 4B, and thus any repetitive explanation regarding the soft actuator will be omitted.

Here, the cool air supplier 600 supplies the air into the soft actuator only in the relaxation state of the first and second soft actuators 10a and 10b.

For example, the cool air supplier 600 may be an air pump or an air bag.

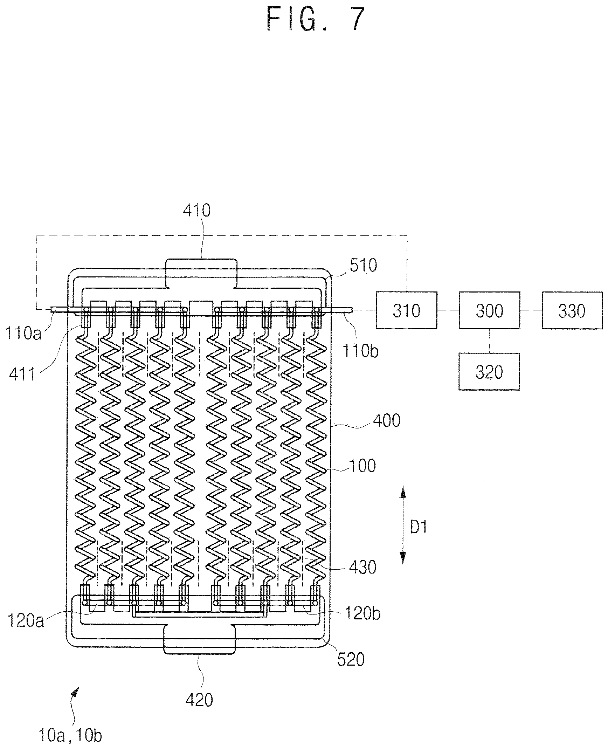

FIG. 7 is a schematic view illustrating one soft actuator of the soft actuator assembly of FIG. 6.

Referring to FIG. 7, each of the soft actuators 10a and 10b according to the present example embodiment, as explained above, may be one of the soft actuator 10 in FIG. 1A, the soft actuator 11 in FIG. 4A, and the soft actuator 12 in FIG. 4B, and for the convenience of explanation, the soft actuator same as the soft actuator 10 in FIG. 1A is exampled.

As the thermal reactive member 100 inside of the soft actuators 10a and 10b is contracted or expanded by the controller 300, the driver itself is contracted and expanded.

The thermal reactive member 100 extends along a direction D1, and the thermal reactive member 100 is contracted or relaxed along the direction D1 according to the change of temperature. In addition, the thermal reactive member 100 may be contracted along the direction D1 in reaction to the heat generated due to the current. In contrast, when the current is not provided and the temperature is decreased, the thermal reactive member 100 may be relaxed along the direction D1.

The thermal reactive member 100 includes the plurality of spring bundles, and the thermal reactive members 100 are arranged in parallel, as explained above.

For example, as illustrated in FIG. 7, a portion 110a of the first end of the thermal reactive members, an opposite portion 110b of the first end t of the thermal reactive members, a portion 120a of the second end of the thermal reactive members, and an opposite portion 120b of the second end of the thermal reactive members are connected with each other. In addition, the portion 120a the second end and the opposite portion 120b the second end may be connected to each other. Then, the current may flow in a series of the portion 110a of the first end, the portion 120a the second end, the opposite portion 120b the second end and the opposite portion 110b of the first end, or flow in the opposite direction thereof.

The thermal reactive member 100 is received by the body 400, and the body 400 has the shape to enclose the thermal reactive member 100 in a whole.

A first fixing portion 510 and a second fixing portion 520 are disposed at the first end and the second end of the body 400, respectively. The first end of the thermal reactive member 100 is fixed to the first fixing portion 510, and the second end of the thermal reactive member 100 is fixed to the second fixing portion 520.

Although not shown in the figure, each of the first and second fixing portions 510 and 520 may include a hole, and thus, the soft actuator 10a and 10b may be connected with other device through the hole.

The body may include various kinds of materials, and may include a material contracted or relaxed with the thermal reactive member at the same time. For example, the body may include a fabric material which is flexible.

A stitch 430 may be formed in the body, to insulate the thermal reactive members with each other. Thus, the thermal reactive members may be prevented from being contacted with each other.

Referring to FIG. 6 again, the soft actuator assembly 2 according to the present example embodiment further includes the controller 300, the power supply 310, the sensor 320 and the power source 330, and the above elements are substantially same as the elements explained referring to FIG. 1A and FIG. 1B, and any repetitive explanation will be omitted.

However, in the present example embodiment, the thermal reactive member 100 is contracted or relaxed according to the supply or interruption of the current. Generally, the relaxation speed of the thermal reactive member 100 is relatively slow when relying on natural cooling, but in the present example embodiment, the response of the relaxation of the thermal reactive member 100 may be improved using the cool air supplier 600 explained below.

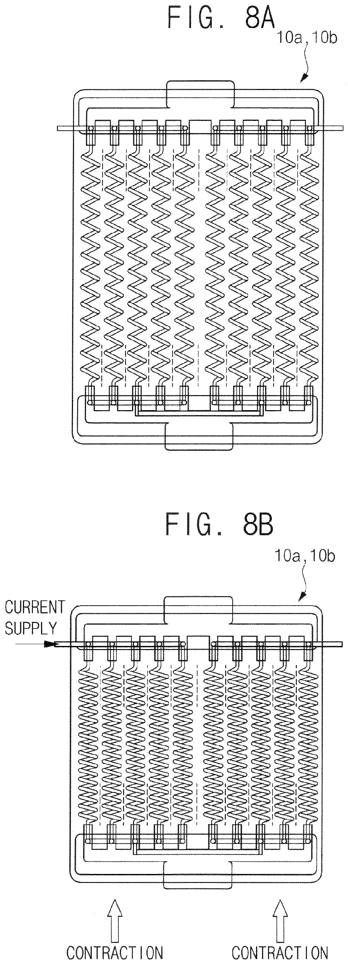

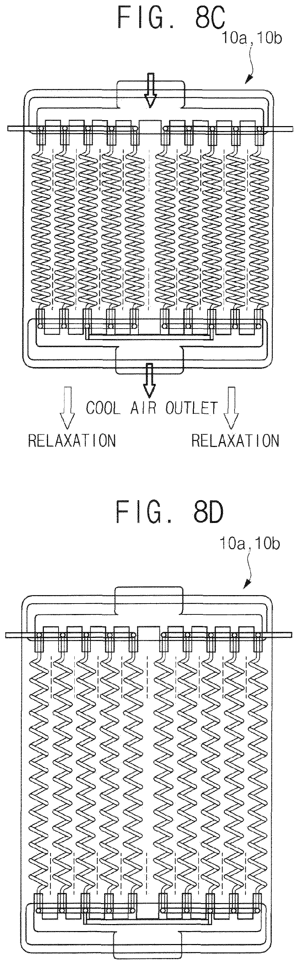

FIG. 8A, FIG. 8B, FIG. 8C and FIG. 8D are schematic views illustrating a contraction motion and a relaxation motion of the soft actuator of FIG. 7.

Referring to FIG. 8A, the soft actuator 10a and 10b is in an initial relaxation state, and the power of the soft actuator 10a and 10b is off and the cool air is not supplied.

Then, in FIG. 8B, the soft actuator 10a and 10b is transformed from the relaxation state to the contraction state, and the power is on and the cool air is not supplied. Here, the current flows into the thermal reactive member 100 to be contracted, and then the body 400 is contracted with the contraction of the thermal reactive member 100.

Then, in FIG. 8C, the soft actuator 10a and 10b is transformed from the contraction state to the relaxation state, and the power is off and the cool air starts to be supplied by the cool air supplier 600.

Finally, in FIG. 8D, the cooling speed of the soft actuator 10a and 10b is returned into the initial relaxation state in a short time, since the cooling speed of the thermal reactive member 100 is increased by the cool air of the cool air supplier 600.

As explained above, the controller 300 of the soft actuator controls the power supply to stop supplying the power to the thermal reactive member 100when the thermal reactive member 100 is changed into the relaxation state. Accordingly, as the power stops and the temperature begins to decrease, the thermal reactive member 100 begins to be relaxed.

In addition, when the cool air is supplied only to the thermal reactive member 100 in the relaxation state, the relaxation speed of the thermal reactive member 100 may be increased. In the present example embodiment, instead of using the active device such as a fan, a blower and so on which requires an additional power, a passive device such as the air pump or the air bag capable of being operated based on an external force generated by the motion of a joint is used, to supply the cool air to the thermal reactive member 100 with the relaxation state, and thus, the relaxation speed of the soft actuator may be more increased.

Hereinafter, the self-supply type cool air supplier 600 is explained in detail.

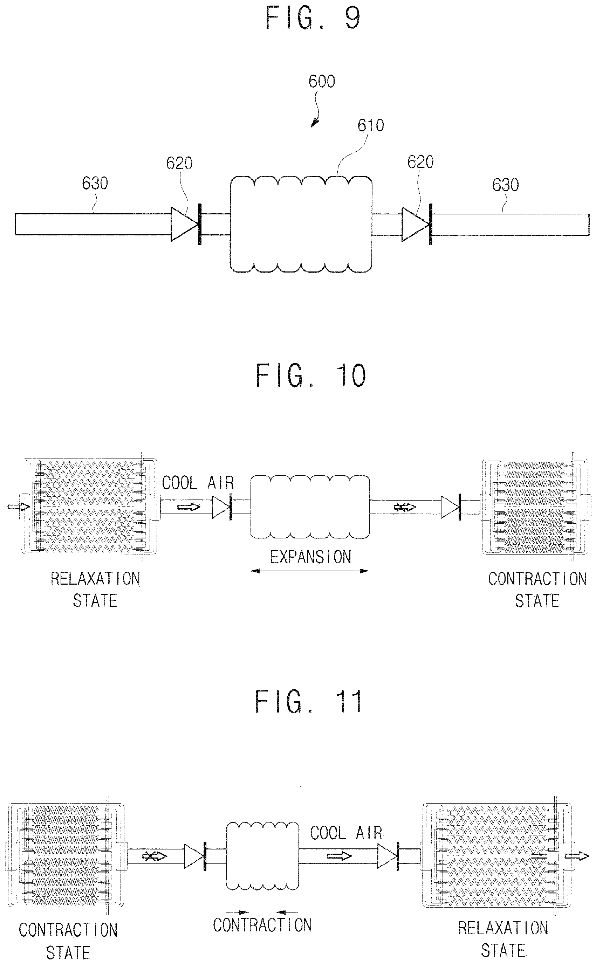

FIG. 9 is a plan view illustrating a cool air supplier of FIG. 6. FIG. 10 is a schematic view illustrating an inflated state of the cool air supplier of the soft actuator of FIG. 6, and FIG. 11 is a schematic view illustrating a contracted state of the cool air supplier of the soft actuator of FIG. 6.

Referring to FIG. 9, the cool air supplier 600 cools the thermal reactive member 100. When the thermal reactive member 100 is in the relaxation state, the cool air supplier 600 supplies the cool air to make direct contact with the thermal reactive member 100, to increase the speed of the relaxation of the thermal reactive member 100. Thus, the problem of the conventional soft actuator in which the relaxation speed is very slow in the natural state may be solved.

The cool air supplier 600 is the self-air-supply device to induce the cool air into the soft actuator 10a and 10b. The operation of the self-air-supply device is explained below referring to FIG. 10 and FIG. 11.

The cool air supplier 600 includes an air pump (or an airbag, 610), a check valve 600 and a cool air path 630. The check valve 600 is disposed at both ends of the air pump 610 to control the flow direction to one way.

The air pump 610 is disposed between the pair of the first and second soft actuators 10a and 10b, and induces the air flow between and inside of the first and second soft actuators 10a and 10b.

The air pump 610 is the passive device operated without the power, and is different from the active device such as the fan, the blower and so on operated with the power.

However, the air pump 610 is expanded and contracted by the external force, and the external force is generated by the motion of the joint.

As illustrated in FIG. 9 and FIG. 10, the air pump 610 includes the check valve 620 disposed at both of the first and second soft actuators 10a and 10b, to control the inlet direction and the outlet direction of the air.

The pair of the check valves 620 controls the air inlet and the air outlet in one way. Thus, the air inflows into the soft actuator only in the relaxation state, of the first and second soft actuators 10a and 10b. Here, the check valve disposed at the soft actuator in the relaxation state of the first and second soft actuators 10a and 10b is open, and the check valve disposed at the soft actuator in the contraction state is closed.

For example, FIG. 10 shows the expanded state of the air pump, and FIG. 11 shows the contracted state of the air pump.

When the air pump is expanded as illustrated in FIG. 10, the air flows into the soft actuator in the relaxation state. The air passing through the first soft actuator 10a increases the cooling speed of the thermal reactive member inside of the actuator, and then the relaxation speed of the first soft actuator 10a is increased. In addition, the cool air does not flow into the second soft actuator 10b in the contraction state, and the contraction speed of the second soft actuator 10b is not decreased.

In contrast, when the air pump is contracted as illustrated in FIG. 11, the air inside of the air pump flows into the soft actuator in the relaxation state. The air passing through the second soft actuator 10b increases the cooling speed of the thermal reactive member inside of the actuator, and then the relaxation speed of the second soft actuator 10b is increased. In addition, the cool air does not go out from the first soft actuator 10a in the contraction state, and the contraction speed of the first soft actuator 10a is not decreased.

The body 400 includes a cool air inlet 410 and a cool air outlet 420. The cool air flows into the body 400 through the cool air inlet 410, and the cool air goes out from the body 400 through the cool air outlet 420. The cool air inlet 410 and the cool air outlet 420 may include a flexible material.

In addition, the cool air inlet 410 and the cool air outlet 420 include an inlet portion 411 and an outlet portion 421 which are opened, respectively. As illustrated in FIG. 6 and FIG. 7, the cool air inlet 410 is formed at a first end of the body which is the upper portion of the fabric, and the cool air outlet 420 is formed at a second end of the body which is the lower portion of the fabric. Then, the openings of the inlet portion 411 and the outlet portion 421 face each other, and thus the air flows smoothly inside of the body.

For example, the inlet portion 411 of the cool air inlet 410 is arranged along the same direction as the thermal reactive member inside of the body, and thus, the cool air flows smoothly into the thermal reactive member and the cooling efficiency may be enhanced.

Hereinafter, a wearable robot (or muscle building robot) having the soft actuator or the soft actuator assembly explained above is explained in detail.

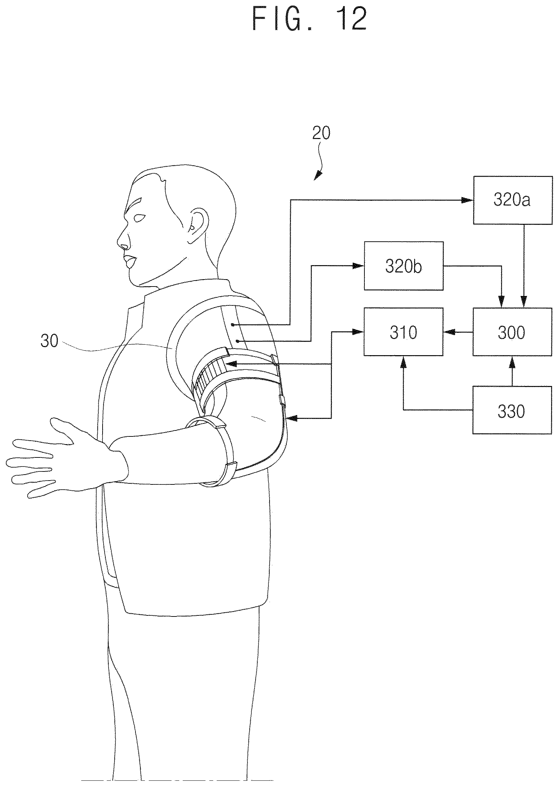

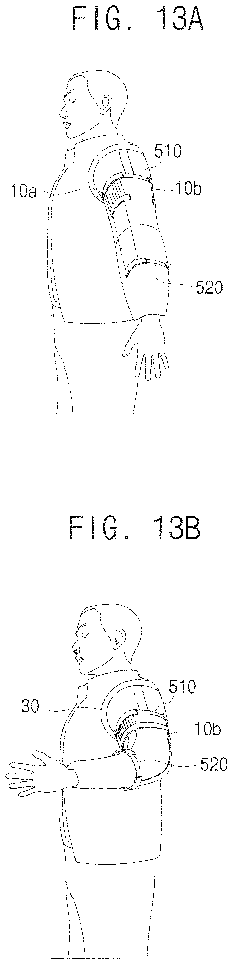

FIG. 12 is a schematic view illustrating a wearable robot according to still another example embodiment of the present invention. FIG. 13A and FIG. 13B are schematic views illustrating an operation state of the wearable robot of FIG. 12.

Referring to FIG. 12, FIG. 13A and FIG. 13B, the wearable robot 20 according to the present example embodiment includes a cloth body 30 and the soft actuator 10a and 10b connected to the cloth body 30.

Here, the pair of the soft actuators 10a and 10b forms the soft actuator assembly 2 in FIG. 6 to FIG. 11, and is equipped to the wearable robot 20. Each soft actuator of the soft actuator assembly 2 may be one of the soft actuators 10a and 10b in FIG. 6 to FIG. 11, the soft actuator 10 in FIG. 1A, the soft actuator 11 in FIG. 4A, and the soft actuator 12 in FIG. 4B.

Further, the soft actuators and the soft actuator assembly are the same as explained above, and thus any repetitive explanation will be omitted. However, hereinafter, for the convenience of explanation, the soft actuator assembly 2 in FIG. 6 to FIG. 11 and the pair of the soft actuators 10a and 10b included in the soft actuator assembly 2 are applied.

The cloth body 30 forms a base of the muscle building robot, and in the present example embodiment the cloth body 30 is exampled as an upper cloth, but not limited thereto. Then, the cloth body 30 may be applied to a lower cloth.

The cloth body 30 includes the inner cover and the outer cover, and the soft actuators 10a and 10b may be connected between the inner cover and the outer cover. In addition, the soft actuator 10a and 10b may be disposed at the position corresponding to the joint of the user in the cloth body 30.

The cloth body 30 includes first and second body fixing portions 510 and 520. The first and second body fixing portions 510 and 520 may be disposed oppositely with respect to the position corresponding to the joint in the cloth body 30.

For example, when the joint is the elbow, the first body fixing portion 510 is disposed to correspond to the brachium, and the second body fixing portion 520 is disposed to correspond to the forearm.

Here, a first end of the soft actuator is fixed to the first body fixing portion 510, and a second end of the soft actuator is fixed to the second body fixing portion 520.

Each of the first and second body fixing portions 510 and 520 may include a band, and the band encloses the arm of the user. Thus, an artificial muscle is attached tightly to the arm of the user, or the first and second body fixing portions 510 and 520 are tightly fixed to the body of the user.

Referring to FIG. 12, the wearable robot 20 includes a sensor 320a and 320b configured to sense the motion of the user. The controller 300 of the wearable robot 20 controls the power supplier 310 to stop supplying the power to the thermal reactive member and to supply the power to the cool air supplier, when the sensor 320a and 320b senses the relaxation motion of the user.

The soft actuators 10a and 10b may be disposed with an antagonistic configuration, for strength support.

Referring to FIG. 13A and FIG. 13B, the wearable robot 20 includes a pair of the first and second soft actuators 10a and 10b.

Thus, when the user wears the wearable robot 20, the first and second soft actuators 10a and 10b may be disposed at an inner side and an outer side of the arm or the leg, so that the first and second soft actuators 10a and 10b face with each other.

Here, the controller 300 of the wearable robot 20 controls the first soft actuator 10 to be contracted and the second soft actuator 10b to be relaxed, when the user is sensed to have the motion, for example the motion of the bending of the arm or the leg.

The controller controls the power supplier 310 to supply the power to the thermal reactive member of the first soft actuator 10a, controls the power supplier 310 to stop supplying the power to the thermal reactive member of the second soft actuator 10b, and controls the power supplier 310 to supply the power to the cool air supplier 600 of the second soft actuator 10b.

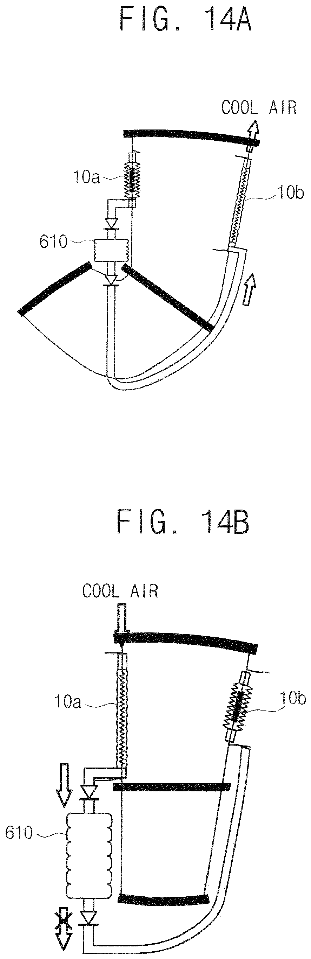

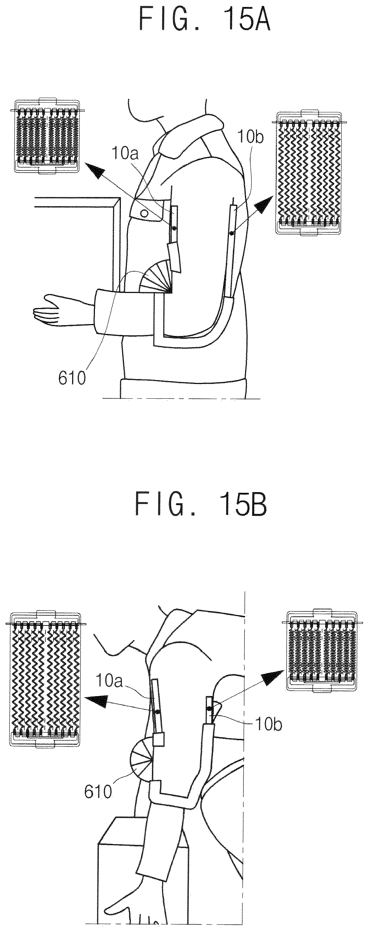

FIG. 14A is a schematic view illustrating a joint flexion state of the wearable robot of FIG. 12, and FIG. 14B is a schematic view illustrating a joint unfolding state of the wearable robot of FIG. 12. FIG. 15A is a schematic view illustrating a joint flexion state with the user wearing the wearable robot of FIG. 12, and FIG. 15B is a schematic view illustrating a joint unfolding state with the user wearing the wearable robot of FIG. 12.

The air pump 610 of the soft actuator assembly 2 in FIG. 9 is expanded and contracted according to a flexed state of the joint and an extended state of the joint, when wearing.

For example, as illustrated in FIG. 15A, the air pump 610 is disposed at the position of the joint. In addition, the first and second soft actuators 10a and 10b may be disposed at the biceps and the triceps, respectively.

Thus, as in FIG. 15A, the joint moves from extended state to the flexed state, the first soft actuator 10a disposed at the biceps is contracted and the second soft actuator 10b disposed at the triceps is relaxed.

Due the flexing motion, the air pump 610 is contracted and the inner air of the air pump flows into the second soft actuator 10b at the triceps. Thus, the cooling speed of the SMA spring of the soft actuator at the triceps is increased, and the relaxation speed is increased.

In contrast, as in FIG. 15B, the joint moves form the flexed state to the extended state, the first soft actuator 10a disposed at the biceps is relaxed and the second soft actuator 10b disposed at the triceps is contracted. Due the extending motion, the air pump 610 is expanded and the external air passes through the first soft actuator 10a at the biceps to flow into the air pump 610. Thus, the cooling speed of the SMA spring of the soft actuator at the biceps is increased, and the relaxation speed is increased.

Accordingly, the soft actuator or the soft actuator assembly explained above may be applied to the wearable robot, and the flexing motion or the extending motion of the joint of the user may be performed more smoothly and easily.

Here, in the present example embodiment, the wearable robot 20 is applied to the biceps and the triceps of the user, but not limited thereto and the same operation mechanism may be applied to other situations. For example, the wearable robot 20 may be combined with various kinds of body parts such as a waist, a knee, an ankle and so, and thus the user's motion such as the extending motion of the joint or the flexing motion of the joint may be performed more smoothly and easily.

According to the present example embodiment, the thermal reactive member implemented as a spring with a fine wire having a fine diameter is used to manufacture the soft actuator and the wearable robot having the soft actuator. Thus, the existing driving force is maintained, the fabric is maintained to have a considerable part of the flexibility in wearing so that awkwardness may not be sensed, and the response performance is significantly improved.

In addition, compared to the conventional complicated manufacturing process, the process for manufacturing the soft actuator may be more simplified and may prevent wastage of materials.

Here, since the SMA spring has a relatively larger power density of the SMA wire and a relatively larger displacement is generated, for example about hundreds of percent (%), the soft actuator may be applied in small size, light weight and noiseless wearable type, and may have a quick response in the relaxation state, due to the cooling system using a self-air supplying type.

Although the exemplary embodiments of the present invention have been described, it is understood that the present invention should not be limited to these exemplary embodiments but various changes and modifications can be made by one ordinary skilled in the art within the spirit and scope of the present invention as hereinafter claimed.

## Claims

1. A soft actuator comprising:
a first spring bundle having a plurality of fine wires, and configured to be capable of being changed between a contraction state and a relaxation state according to a change of temperature;
a first conductive pad having a first connector electrically connected to a first end of the first spring bundle; and
a second conductive pad having a second connector electrically connected to a second end of the first spring bundle,
wherein the first connector is fixed between the first conductive pad and the first spring bundle, and the second connector is fixed between the second conductive pad and the first spring bundle.

2. The soft actuator of claim 1, wherein the first connector is fixed between the first conductive pad and the first spring bundle via sewing, and the second connector is fixed between the second conductive pad and the first spring bundle.

3. The soft actuator of claim 1, wherein each of the fine wires is a shape memory alloy which is driven according to the change of temperature.

4. The soft actuator of claim 1, wherein each of the first and second conductive pads has a foldable conductive material.

5. The soft actuator of claim 1, further comprising:
an outer cover having a flexible material, and having the first spring bundle inside of the outer cover, wherein the first and second conductive pads are fixed to the outer cover.

6. The soft actuator of claim 5, further comprising:
an additional fabric integrally fixed to the first and second connectors, and having a material less flexible than the outer cover.

7. The soft actuator of claim 6, wherein each of the first and second connectors is folded at least one time, and the additional fabric is inserted into and fixed to a folded portion of each of the first and second connectors.

8. The soft actuator of claim 6, wherein the additional fabric comprises an opening portion for an eyelet.

9. The soft actuator of claim 1, wherein the temperature changes according to a current supplied by outside.

10. A soft actuator comprising:
spring bundles having a first spring bundle and a second spring bundle, and configured to be capable of being changed between a contraction state and a relaxation state according to a change of temperature, wherein each of the first and second spring bundles has a plurality of fine wires; and
electrodes electrically connected to the first and second spring bundles, and having a first conductive pad, a middle conductive pad and a second conductive pad,
wherein the first conductive pad has a first connector electrically connected to a first end of the first spring bundle,
wherein the second conductive pad has a second connector electrically connected to a second end of the second spring bundle,
wherein the middle conductive pad has a middle connector electrically connecting the first and second spring bundles with each other.

11. The soft actuator of claim 10, wherein the spring bundles further comprises at least one middle spring bundle disposed between the first spring bundle and the second spring bundle,
wherein the middle spring bundle comprises a plurality of the middle connectors.

12. The soft actuator of claim 10, further comprising:
an external current receiver formed from the first conductive pad, and configured to receive a current from outside to form an electric path between the spring bundles and the electrodes.

13. A wearable robot comprising:
the soft actuator of claim 1;
a sensor configured to sense a motion of a user; and
a controller configured to control the contraction state or the relaxation state of the soft actuator, based on a signal of the sensor,
wherein the soft actuator is disposed at a flexor or an extensor.

14. A soft actuator assembly comprising:
first and second soft actuators configured to be capable of being changed between a contraction state and a relaxation state according to a change of temperature;
a cool air supplier configured to connect the first and second soft actuators with each other to pass through an air, and configured to make the air inflow into a relaxed soft actuator of the first and second soft actuators; and
a controller configured to control the contraction state or the relaxation state of the first and second soft actuators.

15. The soft actuator assembly of claim 14, wherein each of the first and second soft actuators comprises:
a first spring bundle having a plurality of fine wires, and configured to be capable of being changed between the contraction state and the relaxation state according to a change of temperature;
a first conductive pad having a first connector electrically connected to a first end of the first spring bundle; and
a second conductive pad having a second connector electrically connected to a second end of the first spring bundle,
wherein the first connector is fixed between the first conductive pad and the first spring bundle, and the second connector is fixed between the second conductive pad and the first spring bundle.

16. The soft actuator assembly of claim 14, wherein the cool air supplier is operated without a power, and is inflated or contracted by an external force.

17. The soft actuator assembly of claim 14, wherein the cool air supplier comprises a check valve disposed at each of the first and second soft actuators to control a flow direction of the air,
wherein the check valve at the relaxed soft actuator of the first and second soft actuators is open, and the check valve at a contracted soft actuator of the first and second soft actuators is closed.

18. The soft actuator assembly of claim 14, wherein each of the first and second soft actuators comprises a thermal reactive member configured to be capable of being changed between the contraction state and the relaxation state according to a change of temperature,
wherein a cool air for cooling the thermal reactive member makes direct contact with the thermal reactive member to increase a relaxation speed of the thermal reactive member, when the thermal reactive member is in the relaxation state.

19. A wearable robot comprising:
the soft actuator assembly of claim 14,
wherein the cool air supplier is disposed at a joint, the first soft actuator is disposed at a flexor and a second soft actuator is disposed at an extensor.

20. The wearable robot of claim 19, wherein the first soft actuator is contracted and the second soft actuator is relaxed, and then the cool air supplier is contracted to move an inner air to the second soft actuator, when the joint moves from an extended state to a flexed state,
wherein the first soft actuator is relaxed and the second soft actuator is contracted, and then the cool air supplier is inflated to move an external air to pass through the first soft actuator into the second soft actuator, when the joint moves from the flexed state to the extended state.
